# EUROPEAN PATENT APPLICATION

(11) **EP 2 388 022 A1**
(43) Date of publication of application: **23.11.2011**
(21) Application number: 10005216.6
(22) Date of filing: 19.05.2010
(51) Int. Cl.: A61L 27/38, A61L 27/52, D04B 21/16, C12N 5/071

(54) **Cell culture and process for preparing the same**

(71) Applicant: Stichting Dutch Polymer Institute, 5612 AB Eindhoven (NL)
(72) Inventor: The designation of the inventor has not yet been filed
(74) Representative: Renkema, Jaap

(57) **Abstract**

The invention relates to a cell culture comprising a three-dimensional scaffold woven or knitted from fibers. The fibers include a cell-embedded fiber In which living cells are embedded. A process is also provided for making a cell culture comprising a three-dimensional scaffold woven or knitted from fibers. The process comprises the steps of: (a) providing a cell-embedded fiber in which living cells are embedded and (b) using the cell-embedded fiber to weave or knit the three-dimensional scaffold of the cell culture; or treating the three-dimensional scaffold of the cell culture to modify the woven or knitted fibers into cell-embedded fibers.

## Description

The present Invention relates to a cell culture such as a tissue restoration implant, comprising a three-dimensional woven or knitted scaffold. The present Invention also relates to a process for preparing such cell culture.

Tissue engineering, the multidisciplinary enterprise involving the development of biological substitutes that restore, maintain, or improve tissue function or a whole organ is a highly diverse and evolving discipline. Most broadly, tissue engineering involves the creation of scaffolds to provide mechanical support and host living cells.

One approach to tissue engineering can be categorized as build-to-stock manufacturing techniques. In this category, the production plans are based on a build-ahead strategy, where a stock of different sizes and dimensions of products Is prepared ahead of time, and stored until needed. One such approach Involves the use of tissue scaffolds that are biological in origin, *i.e.* decellularized xenogeneic, allogeneic, or cadaveric materials. In such cases, an artificial tissue or organ Is prepared from material taken from another organism, which has been decellularized by perfusion, then loaded with the appropriate cell culture, and cultivated under specific conditions to promote the desired proliferation. While this offers a direct route to a biomorphic system, it requires a stockpile of organs and tissues of a broad size range to fit to recipient. Of course, there are also risks Involved in infection and pathogen transmission, plus immunoresponsive/biocompatibility issues.

In another approach, a rapid prototyping of a scaffold is enabled using a computer-assisted design based technologies. This technology involves preparing a CAD file, outlining the dimensions of the prototype, which Is then translated into a series of commands to build up the system. Stereolithography and ink-Jet 3D printing are examples of this approach that permit a high degree of spatial resolution and control on the order of tens of microns. This technology has a drawback that the throughput is typically low.

In another approach, textile reinforced composites are used for tissue engineering. W02006/113641A1 discloses a tissue restoration implant adapted for use with a pro-determined tissue comprising a three-dimensional fiber scaffold comprising at least three systems of fibers and one or more cells that can develop into the pre-determined tissue. The fiber scaffold or one or more of the fiber systems provide a characteristic that functions to restore the pre-determined tissue upon implantation, The three-dimensional fiber scaffold comprises different types of woven fiber systems. The cells may be present In a resin or gel matrix which occupies the pore space of the fiber scaffold.

Wan, et al, J. Biomed. Mater. Res., Part A, 2004, 71A, 586-595 discloses encapsulation of biologics in self-assembled fibers as biostructural units for tissue engineering. Use of fibers incorporating biological factors for making nonwoven biostructural units is mentioned. However, the publication is silent on a three-dimensional woven or knitted scaffold.

Although the known cell cultures are satisfactory for some applications, there is an increased need in the art for cell cultures which can be used in a wide range of situations.

An objective of the present Invention is to provide an Improved cell culture, comprising a three-dimensional woven or knitted scaffold where the above-mentioned and/or other needs in the art are met.

Accordingly, the present Invention provides a cell culture comprising a three-dimensional scaffold woven or knitted from fibers, wherein the fibers Include a cell-embedded fiber in which living cells are embedded.

A cell culture is understood to mean any article In which cells are cultured. It may be a tissue restoration implant, an artificial organ or a bioreactor or the like.

Within the context of the present invention, a fiber formed of a material within which living cells are Incorporated is called a cell-embedded fiber. A hollow fiber having cells which are present only In its central cavity is excluded from the definition of a cell-embedded fiber.

Within the context of the present invention, a fiber is understood to mean an elongated body having length dimension much greater than its cross section dimensions. Typically, the largest cross section dimension (diameter if the cross section is substantially circular) is sufficiently large enough to host living cells but also thin enough to be flexible processable, and not inhibit respiration of the cells. Preferably, the largest cross section dimension is greater than 10 µm, and less than 1,000 µm. Fibers include both staple fibers and much longer filament fibers. Fibers can be monofilament or multifilament, and can be of any shape or cross-section. The cross-section can vary along the length of fiber. Fibers may also be hollow.

Weaving or knitting of fibers is herein understood to mean interlacing fibers to construct an article.

According to the present invention, the scaffold has an Intrinsically open structure woven or knitted from cell-embedded fibers enabling an easy diffusion of substances to and from the cells. This is critically important since the cells require substances such as oxygen for culturing. Substances such as oxygen and nutrients must be able to be transported to the cells and carbon dioxide and metabolites must be transported out from the cells. The distance between the cells and the surrounding environment in which these substances are present plays an important role in the transportation. In the conventionally fabricated structures in which the cells are embedded in a matrix, transport of substances (*e.g.* oxygen) to and from the cells is restricted by the matrix, and hence the depth in which cells may be viably cultured In the matrix is also restricted. According to the present Invention, the fibers are woven or knitted to form the scaffold and are not embedded in a matrix. The fibers are thus in a direct contact with the environment in which substances such as oxygen are present. The substances need to travel through only a small distance to reach the living cells, because of the small cross-section of the fibers. The transportation of substances is thus inhibited to a much less degree compared to the conventional scaffold, irrespective of the winding or knitting pattern of the fibers in the scaffold.

Furthermore, the inventors have realized that the embedding of the living cells into fibers and forming a woven or knitted structure using these fibers would allow precise control of the three-dimensional positioning of the cells, since the positioning of the fibers can be well controlled with weaving or knitting. Well-established technology of weaving or knitting enables precise positioning of fibers in which cells are embedded Into specific locations within the scaffold, with good control of relative positioning. In conventional structures where the cells are in the matrix or in a non-woven structure, the cells are randomly dispersed without a precise control, since the positions of the carriers of the cells are not precisely controllable. Consequently, more complex ordered architectures are possible according to the invention. For example, some fibers with a specific type of cells can be guided through the structure to encourage angioneogenesis in a desirable pattern, while other fibers can be used to Impart enhanced mechanical stability, or release of endostatins, or other functions. Weaving or knitting also allows for different components to be Incorporated in different directions, allowing for anisotropic behavior from the finished product, including directionally-oriented elasticity and/or stiffness. Furthermore, control over the weave or knit density is also advantageous in designing tough materials.

The cells may be of any type which exhibit attachment and ingrowth and are suitable for the Intended target tissue, tissues, and/or envisioned location of implantation for the three-dimensional fiber scaffold. For example, the cells may be mammalian cells, such as human cells. Cells may be primary cells, undifferentiated progenitor cells, chondrocytes, bone-precursor cells, stem cells, synovial cells, umbilical cord cells, cord blood cells, muscle stem cells, adipose cells, preadipocytes, hematopoietic stem cells, mesenchymal stem cells, cells of the periosteum, or perichondrium tissue, stromal cells, embryonic stem cells, germ cells, and combinations of any of the foregoing.

The cell culture may be an artificial blood vessel, such as a capillary.

In some embodiments of the present invention, the cell-embedded fiber comprises a hydrogel in which the cells are embedded. The cells are incorporated In the network of polymer of the hydrogel. The material for the hydrogel is gellable, resulting after gelling in either a chemically crosslinked system such as In case of calcium alginate or a physically crosslinked system such as in case of gelatin. The system consists of a network of solubilized or swollen polymeric chains that are either physically entangled with each other or chemically crosslinked to each other in such a way that the system has a non-zero shear modulus.

Preferred examples of a hydrogel Include polyuronic acids, such as alginates. For example, if sodium alginate solutions are injected into a bath containing calcium ions, the solution will undergo a metathesis process, whereby sodium is replaced by calcium, yielding an lonically-crosslinked hydrogel. Alginate Is a linear copolymer composed (1,4)-linked *β*-D-mannuronic acid and α-L-guluronic acid, and can be easily transformed into a gel by binding the guluronic acids with a divalent cation, such as calcium (see: Biomedical Microdevices 2007, 9(6) 855-862),

Still other preferred hydrogels are non-crosslinked polyacrylamides, polyacrylic acid and its salt and their block copolymers. These polymers are soluble at lower pH values and are insoluble at higher pH values.

Still other preferred hydrogels are selected proteins which are gelatin and/or collagen and which is/are added as aqueous solution to the liquid of polymerisable monomers. These proteins are classic examples of materials capable of forming thermally reversible hydrogels, where polymer chains become entangled, but which can be reversed by heating.

The cell-embedded fibers of the scaffold may comprise additional functional components such as growth factors. Further examples include cytokines, chemokines, collagens, gelatin, laminin, fibronectin, thrombin, lipids, cartilage oligomeric protein (COMP), thrombospondin, fibrin, fibrinogen, Matrix-GLA (glycine-leucine-alanine) protein, vascular endothelial growth factor, chondrocalcin, tenascin, a mineral, an Arginine-Glycine-Aspartic Acid (RGD) peptide or RGD-peptide containing molecule, elastin, hyaluronic acid, a glycosaminoglycan, a proteoglycan, water and an electrolyte solution.

In some embodiments of the present invention, the cell-embedded fiber further comprises anisotropic particles such as carbon nanotubes. These anisotropic particles may Increase the mechanical properties of the cell-embedded fiber by being suitably oriented.

In some embodiments of the present invention, the cell-embedded fiber further comprises agents to improve its mechanical strength. Such agents include stiffening agents and elastomers. This Improves the handling of the cell-embedded fiber. The scaffold also becomes more robust.

In some embodiments of the present invention, the cell-embedded fiber comprises a core and a sheath surrounding the core, the sheath comprising the hydrogel In which the cells are embedded. The core is a fiber which is preferably made of a biocompatible material. Examples of suitable core material Include polypropylene, polyester such as polyglycolic acid (PGA), polylactic acid (PLA), poly(lactidelglycolide), polycaprolactone,, polytetrafluoroethylene (PTFE), expanded PTFE (ePTFE), polyethylene, polyurethane, polyamide, nylon, polyetheretherketone (PEEK), polysulfone, a cellulose and its derivatives, fiberglass, an acrylic, polyvinyl alcohol, carbon, ceramic, a metal, polydioxanone, polyoxalate, a polyanhydride, a poly(phosphoester), catgut suture, collagen, slik, chitin, chitosan, hydroxyapatite, bioabsorbable calcium phosphate, hyaluronic acid, elastin, and combinations thereof. Particularly preferred is poly(lactide/glycolide). Cellulose derivatives include cellulose that has been functionalized, such as methyl cellulose, or hydroxypropylmethyl cellulose, and cellulose that has been partially degraded or oxidized In a controlled manner. The core preferably has a strength which facilitates the weaving or knitting of the cell-embedded fiber. Preferably, the core fiber of the cell-embedded fiber has a tensile strength of at least 1 MPa. This embodiment has a further advantage that the amount of the living cells used may be saved.

In some embodiments of the present invention, the cell-embedded fiber Is hollow. The cavity of the fiber may contain additional living cells or other desired components.

In some embodiments of the present Invention, the woven or knitted fibers further include reinforcement fibers having a tensile strength of at least 1 MPa. Preferably, the reinforcement fibers have a tensile strength of at least 500 MPa, more preferably 1000 MPa, more preferably 1500 MPa, more preferably 2000 MPa, more preferably 3000 MPa. There is no upper limit for the tensile strength of reinforcement fiber suitable for use in the present invention. A very strong known fiber made from ultrahigh molecular weight polyethylene is known to have a tenacity of as high as 3500 MPa. Suitable materials for the reinforcement fibers include the materials mentioned for the core material in the embodiment where the fiber comprises a core and a sheath. The reinforcement fibers may first be twisted or assembled in other known suitable ways with the cell-embedded fibers to form a yarn before being woven or knitted to form the scaffold, This allows an easy weaving or knitting, since the yarns to be woven or knitted are already reinforced, and thus handling of the yarns during a possibly complex weaving or knitting process is easier. For example, the cell-embedded fibers may be wound around one or more reinforcement fibers and a resulting yarn may be woven or knitted. Alternatively, the reinforcement fibers and the cell-embedded fibers may be made into separate yarns and be woven or knitted to form the scaffold. It will be appreciated that both ways of assembly of the cell-embedded fibers and the reinforcement fibers may be combined in one embodiment.

In some embodiments of the present Invention, the woven or knitted fibers Include a first group of cell-embedded fibers and a second group of cell-embedded fibers, wherein the cells in the first group of cell-embedded fibers are different from the second group of cell-embedded fibers. This allows the cell culture to contain more than one type of cells and thus the cell culture according to the present invention can be formed to have different functions at different locations. For example, fibers comprising HepG2 may be concentrically wound around several fibers comprising HEK-293 cells representing blood vessels. Further, fibers comprising L-929 cells may be wound over the fibers comprising HepG2 wound around the central fibers comprising HEK-293 cells. This is an example of connecting and culturing different cell types into a single component. The advantage to this is that it allows for different tissue types to be Integrated through a controlled structural gradient.

According to an yet further aspect of the Invention, a process for making a cell culture comprising a three-dimensional scaffold is provided. The process comprises the steps of providing a cell-embedded fiber In which living cells are embedded and using the cell-embedded fiber to weave or knit the three-dimensional scaffold of the implant; or treating the three-dimensional scaffold of the implant to modify the woven or knitted fibers into cell-embedded fibers.

In some embodiments of the present invention where a cell-embedded fiber is used to weave or knit a three-dimensional scaffold of the implant, the step of providing the cell-embedded fiber comprises extruding a curable liquid comprising living cells into a curing environment to form the cell-embedded fiber. This process is advantageous, in that the cell-embedded fiber is provided in just one step, without requiring to provide other fibers.

In some embodiments of the present invention, the curable liquid comprises a solution of a hydrogel in a fluid state. The curing environment comprises a liquid medium which gellates the curable liquid.

In some embodiments of the present invention where a cell-embedded fiber Is used to weave or knit a three-dimensional scaffold of the implant, the step of providing the cell-embedded fiber comprises providing a core fiber, soaking the core fiber In a curable liquid and treating the core fiber in a curing environment comprising living cells to cure the curable liquid around the core fiber to form a sheath In which living cells are embedded.

In some embodiments of the present invention, the core fiber is made of a degradable material and the step of providing the cell-embedded fiber comprises degrading the core fiber to form a hollow fiber. An example of suitable degradable material Is hyaluronic acid.

In some embodiments of the present invention where a three-dimensional scaffold of the implant is treated, the process comprises the steps of soaking the three-dimensional scaffold of the implant in a curable liquid and treating the scaffold in a curing environment comprising living cells to cure the curable liquid around the fibers to form a sheath In which living cells are embedded.

In some embodiments of the present invention, the three-dimensional scaffold is formed from the fibers by winding the fibers around a mandrel and removing the mandrel. In this way, a tube-shaped scaffold is formed easily. The material for the mandrel is not critical, but a suitable example is PTFE.

According to an yet further aspect of the present invention, use of fibers In which cells are embedded for preparing a cell culture is provided.

The present Invention will be illustrated with reference to the following non-limiting examples.

### Example 1

A sodium alginate solution (1% w/v in RPMI 1640 cell culture medium) containing 2 x 10⁶ cells.mL⁻¹ of living L929 mouse fibroblast cells and 0.1% w/v of brilliant blue G dye for improved visualization was prepared.

Dropjet MJ-E-130 continuous printing system from Microdrop Technologies GmbH (Norderstedt, Germany) was fitted with a 100 µm Inner diameter nozzle. The printing system was loaded with 10 mL of the sodium alginate solution. The solution was processed using back pressure of 380 kPa, where it was dispensed downwards into a reservoir conntaining 50 mL of 15 wt% calcium chloride solution, which was sitting 5 cm below the nozzle. The sodium alginate solution reacted with the calcium chloride solution and fibers containing L929 mouse fibroblast cells were formed. The fibers were wound up so that they may be woven.

### Example 2

A surgical-grade poly(lactidelglycoilds)-based suture was soaked in 2% calcium chloride aqueous solution and was wrung out to remove excess liquid. The suture was passed through a gellable cell suspension. The gellable cell suspension was comprised of a solution of sodium alginate (1 wt%) in cell culture medium RPMI 1640 containing 2 x 10⁶ of cells-mL⁻¹ of cell L929. The fibers were then rinsed once with pure medium to remove any excess of non-crosslinked sodium alginate solution. The cell-loaded suture was woven around a removable mandrel,

### Example 3

Fibers containing cell HepG2 and fibers containing HEK-293 were prepared in the similar manner as in example 2. The fibers containing L929 and the fibers containing HepG2 were woven around the fibers containing HEK-293 using manual plain weaving technique. Cell viability, as defined by number of living cells divided by the total number of cells, was measured by optical microscopy after 0, 2, 4, 6 and 8 days after incubation. Cellular viability remained above 0.9 after 8 days.

### Example 4

A cotton gauze was wrapped around a polypropylene mandrel, which is then soaked in a CaCI₂ bath. The cotton gauze was wrung out to remove excess solution, and then was Immersed in an alginate solution containing living L929 cells. Crosslinking of the alginate was induced and a hydrogel structure was formed around the mandrel. The mandrel was removed to yield a hydrogel structure seeded with living L929 cells.

## Claims

1. A cell culture comprising a three-dimensional scaffold woven or knitted from fibers, wherein the fibers include a cell-embedded fiber In which living cells are embedded.

2. The cell culture according to claim 1, wherein the cell-embedded fiber comprises a hydrogel in which the cells are embedded.

3. The cell culture according to claim 1 or 2, wherein the cell-embedded fiber comprises a core and a sheath surrounding the core, the sheath comprising the hydrogel in which the cells are embedded.

4. The cell culture according to any one of claims 1-3, wherein the cell-embedded fiber is hollow.

5. The cell culture according to any one of claims 1-4, wherein the woven or knitted fibers further include reinforcement fibers having a tenacity of at least 1 MPa.

6. The cell culture according to any one of claims 1-5, wherein the woven or knitted fibers include at least two cell-embedded fibers where the types of the embedded cells are different.

7. A process for making a cell culture comprising a three-dimensional scaffold woven or knitted from fibers, comprising the steps of:
- (a) providing a cell-embedded fiber in which living cells are embedded and (b) using the cell-embedded fiber to weave or knit the three-dimensional scaffold of the cell culture; or
- treating the three-dimensional scaffold of the cell culture to modify the woven or knitted fibers into cell-embedded fibers.

8. The process according to claim 7, wherein step (a) comprises extruding a curable liquid comprising living cells into a curing environment to cure the curable liquid to form the cell-embedded fiber.

9. The process according to claim 8, wherein the curable liquid comprises a solution of a hydrogel In a fluid sol state and the curing environment comprises a liquid medium which gelates the curable liquid.

10. The process according to claim 7, wherein step (a) comprises providing a core fiber, soaking the core fiber in a curable liquid and treating the core fibers In a curing environment comprising living cells to cure the curable liquid around the core fibers to form a sheath In which living cells are embedded,

11. The process according to claim 10, wherein the core fibers are degradable and the core fibers are degraded to form hollow fibers.

12. The process according to claim 7, wherein step of treating the three-dimensional scaffold of the cell culture comprises soaking the three-dimensional scaffold of the cell culture In a curable liquid and treating the scaffold in a curing environment comprising living cells to cure the curable liquid around the fibers to form a sheath in which living cells are embedded.

13. The process according to any one of claims 8-12, wherein the curable liquid comprises a sodium alginate solution and the curing environment comprises a calcium chloride solution.

14. The process according to any one of claims 7-13, wherein the three-dimensional scaffold is formed from the fibers by winding the fibers around a mandrel and removing the mandrel.

15. Use of cell-embedded fibers for preparing a tissue restoration implant.
